# FASCICULE DE BREVET EUROPEEN

(11) **EP 3 775 802 B1**
(45) Date de publication et mention de la délivrance du brevet: **27.04.2022**
(21) Numéro de dépôt: 19720945.5
(22) Date de dépôt: 28.03.2019
(51) Int. Cl.: G01J 1/08, G01J 1/42

(54) **IMAGEUR TÉRAHERTZ À CHAMP PROCHE**
NAHFELD-TERAHERTZBILDGEBUNGSVORRICHTUNG
NEAR-FIELD TERAHERTZ IMAGING DEVICE

(30) Priorité: 28.03.2018 FR 1852688
(43) Date de publication de la demande: 17.02.2021
(73) Titulaire: Tihive, 38240 Meylan (FR)
(72) Inventeur: SHERRY, Hani, 38000 Grenoble (FR)
(74) Mandataire: de Jong, Jean Jacques
(86) Numéro de dépôt international: PCT/FR2019/050720
(87) Numéro de publication internationale: WO 2019/186074

(56) Documents cités:
- FR-A1- 3 035 499
- JANUSZ GRZYB ET AL: "Solid-State Terahertz Superresolution Imaging Device in 130-nm SiGe BiCMOS Technology", IEEE TRANSACTIONS ON MICROWAVE THEORY AND TECHNIQUES, vol. 65, no. 11, 1 novembre 2017 (2017-11-01), pages 4357-4372, XP055527558, USA ISSN: 0018-9480, DOI: 10.1109/TMTT.2017.2684120
- SENGUPTA KAUSHIK ET AL: "Silicon Integrated 280 GHz Imaging Chipset With 4$\times$ 4 SiGe Receiver Array and CMOS Source", IEEE TRANSACTIONS ON TERAHERTZ SCIENCE AND TECHNOLOGY, IEEE, PISCATAWAY, NJ, USA, vol. 5, no. 3, 1 mai 2015 (2015-05-01), pages 427-437, XP011579783, ISSN: 2156-342X, DOI: 10.1109/TTHZ.2015.2414826 [extrait le 2015-04-29] & KAUSHIK SENGUPTA ET AL: "Distributed active radiation for THz signal generation", SOLID-STATE CIRCUITS CONFERENCE DIGEST OF TECHNICAL PAPERS (ISSCC), 2011 IEEE INTERNATIONAL, IEEE, 20 février 2011 (2011-02-20), pages 288-289, XP032013741, DOI: 10.1109/ISSCC.2011.5746322 ISBN: 978-1-61284-303-2
- PFEIFFER ULLRICH R ET AL: "A 0.53 THz Reconfigurable Source Module With Up to 1 mW Radiated Power for Diffuse Illumination in Terahertz Imaging Applications", IEEE JOURNAL OF SOLID-STATE CIRCUITS, IEEE SERVICE CENTER, PISCATAWAY, NJ, USA, vol. 49, no. 12, 1 décembre 2014 (2014-12-01), pages 2938-2950, XP011564903, ISSN: 0018-9200, DOI: 10.1109/JSSC.2014.2358570 [extrait le 2014-11-20]

## Description

### Domaine technique

L'invention concerne les techniques d'imagerie de proximité, utilisant notamment des sondes du domaine térahertz à placer en contact avec un objet à analyser.

### Arrière-plan

Le domaine des ondes térahertz (THz) est compris entre les ondes millimétriques et le rayonnement visible. On admet que le domaine térahertz s'étend en fréquence d'environ 300 GHz à quelques THz. Dans ce domaine, les ondes ont des propriétés à la fois du domaine radiofréquence et du domaine optique - elles peuvent en particulier être émises et reçues par des antennes, et être focalisées par des systèmes optiques, par exemple des lentilles en silicium.

Les ondes THz ont la propriété de traverser certains objets sans avoir la nocivité des rayons-X. En imagerie médicale, on les utilise, par exemple, pour détecter des tissus cancéreux, du fait que ces tissus ont des propriétés d'absorption et réflexion différentes des tissus sains dans le domaine THz.

L'article ["Use of a handheld terahertz pulsed imaging device to differentiate benign and malignant breast tissue", Maarten R. Grootendorst et al., Vol. 8, No. 6, 1 Jun 2017, Biomédical Optics Express 2932] décrit une sonde portative conçue pour être glissée au contact de la peau d'un patient et analyser celle-ci par réflexion d'ondes, à la façon d'une sonde d'échographie.

Les ondes THz sont mises en œuvre dans la sonde par l'intermédiaire d'impulsions laser femtoseconde générées hors de la sonde et guidées le long de fibres optiques vers un émetteur/récepteur photoconducteur placé dans la sonde. Des impulsions résultantes de 0,1 à 1,8 THz sont ensuite guidées par un miroir oscillant entre l'émetteur/récepteur et une fenêtre de quartz présente à l'extrémité de la sonde, pour balayer pas à pas 26 pixels dans une zone de 15 x 2 mm à une fréquence de 4 Hz. A chaque pas du balayage, des impulsions THz réfléchies sont renvoyées par le pixel correspondant vers le récepteur.

Une telle sonde portative met en œuvre des technologies optiques complexes et coûteuses. En outre, le pas des pixels, de l'ordre de 0,6 mm, offre une résolution relativement basse. Cette résolution est contrainte par la précision du mécanisme entraînant le miroir et la longueur d'onde relativement importante des ondes THz. Le pas de 0,6 mm des pixels correspond sensiblement à la limite de diffraction d'Abbe dans l'air pour la fréquence la plus basse des impulsions utilisées, ici 0,1 THz et une longueur d'onde de 1,2 mm.

Un tel système requiert ainsi un équipement encombrant et coûteux pour mettre en œuvre un capteur d'image de seulement 15 x 2 mm, l'essentiel de l'encombrement étant constitué par l'équipement de mise en œuvre des faisceaux laser nécessaires.

On est récemment parvenu à réaliser des récepteurs et des émetteurs THz à l'aide de technologies des semi-conducteurs, qui sont entièrement exploitables par des circuits électroniques intégrés sur les mêmes puces.

On trouve ainsi des récepteurs THz regroupés dans une matrice sur une puce en semi-conducteur pour former un capteur d'image compact. Par exemple, l'article ["A 1 k-Pixel Video Camera for 0.7-1.1 Terahertz Imaging Applications in 65-nm CMOS", Richard Al Hadi, Hani Sherry, et al., IEEE Journal of Solid-State Circuits, VOL. 47, NO. 12, December 2012] décrit un capteur d'image formé de récepteurs THz entièrement réalisés en technologie CMOS 65 nm. Les récepteurs parviennent à traiter des signaux de fréquence supérieure à la fréquence de fonctionnement des transistors grâce à l'utilisation d'éléments passifs et de configurations où les transistors sont moins limités en fréquence (transistors montés en source commune).

On est également parvenu à concevoir des émetteurs THz intégrables en technologie des semi-conducteurs, notamment CMOS. Une difficulté pour les émetteurs était de produire des signaux THz de fréquence supérieure à la fréquence de fonctionnement des transistors. Cette difficulté a été levée en utilisant des oscillateurs dits harmoniques. Un tel oscillateur fonctionne à une fréquence compatible avec la technologie et produit des harmoniques exploitables dans le domaine THz. Le brevet US9083324 décrit un tel oscillateur.

De plus amples informations sur des récepteurs et émetteurs THz intégrables peuvent être obtenues dans les thèses de Hani Sherry et Richard Al Hadi soutenues à l'université de Wuppertal en 2013.

Malgré la faisabilité démontrée d'une intégration de composants THz sur des puces de semi-conducteur, on n'est pas parvenu à proposer des sondes de proximité compactes pouvant remplacer celle décrite dans l'article susmentionné de Biomédical Optics Express, par exemple.

Le brevet US9464933 décrit un imageur THz en champ proche comprenant une matrice de capteurs. Chaque capteur comprend une ligne de transmission couplée entre un oscillateur et un circuit de détection. L'oscillateur génère un champ qui est modifié par la proximité d'un objet à analyser. La modification est traduite par des variations d'impédance sur la ligne de transmission, mesurées par le circuit de détection.

### Résumé

On prévoit de façon générale un capteur pour système d'imagerie térahertz, comprenant une matrice de récepteurs d'un rayonnement térahertz ; et une matrice d'émetteurs du rayonnement térahertz ayant le même pas que la matrice de récepteurs, disposée entre la matrice de récepteurs et une zone d'analyse située dans le champ proche des émetteurs, et configurée de manière que chaque émetteur émette une onde à la fois vers la zone d'analyse et vers un récepteur respectif de la matrice de récepteurs.

Le capteur peut comprendre en outre un premier substrat plan en matériau semi-conducteur transparent aux rayonnements térahertz, ayant une face active sur laquelle sont réalisés les récepteurs dans une technologie des semi-conducteurs ; et un deuxième substrat plan en matériau semi-conducteur transparent aux rayonnements térahertz, ayant une face active sur laquelle sont réalisés les émetteurs dans une technologie des semi-conducteurs.

Le capteur peut comprendre en outre un circuit de commande configuré pour activer en séquence chaque émetteur avec son récepteur respectif.

La face active du deuxième substrat peut être tournée vers la zone d'analyse, et une face arrière du deuxième substrat tournée vers le premier substrat.

La face active du premier substrat peut être tournée du côté opposé au deuxième substrat, et une face arrière du premier substrat tournée vers le deuxième substrat.

Les premier et deuxième substrats peuvent être séparés l'un de l'autre par une couche ayant un indice de réfraction plus faible que celui des substrats.

Le pas des matrices peut être au moins égal à la moitié de la longueur d'onde du rayonnement à l'intérieur des substrats et chaque substrat peut avoir une épaisseur au plus égale à la moitié de la longueur d'onde du rayonnement à l'intérieur du substrat.

Les récepteurs et les émetteurs peuvent avoir une configuration hexagonale et être agencés selon des matrices en nid d'abeille.

Chaque récepteur et émetteur peut comprendre une antenne annulaire formée dans un niveau de métal de la face active, la circonférence moyenne de l'antenne étant au moins égale à la moitié de la longueur d'onde du rayonnement térahertz à l'intérieur du substrat ; et un anneau de garde entourant l'antenne à la périphérie du récepteur ou émetteur, formé à partir de plages métalliques empilées sur plusieurs niveaux de métal.

L'anneau de garde peut comprendre des plages métalliques structurées pour former une cavité abritant des pistes conductrices et des composants électroniques servant à exploiter les récepteurs et émetteurs.

### Description sommaire des dessins

Des modes de réalisation seront exposés dans la description suivante, faite à titre non limitatif en relation avec les figures jointes parmi lesquelles :
- la figure 1 est une vue en coupe partielle et schématique d'un mode de réalisation de capteur d'image térahertz à champ proche compact ;
- la figure 2 représente une vue de dessus d'un mode de réalisation de pixels hexagonaux réalisés dans une technologie des semi-conducteurs ; et
- la figure 3 représente une vue en coupe d'un exemple de configuration des pixels de la figure 2.

### Description de modes de réalisation

On propose ci-après de combiner une matrice de récepteurs térahertz et une matrice d'émetteurs térahertz, chacune réalisable sur une puce, dans un imageur compact à appliquer contre un objet à analyser. On utilise pour cela les émetteurs et récepteurs en mode champ proche, c'est-à-dire à une distance suffisamment faible, inférieure à la longueur d'onde, pour exploiter le couplage magnétique entre éléments.

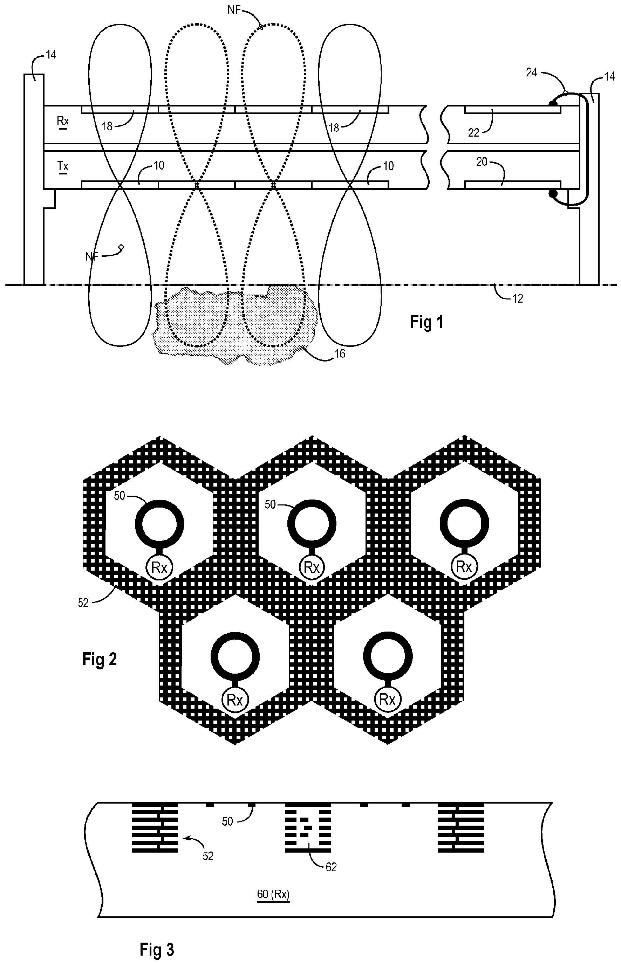

La figure 1 illustre schématiquement un mode de réalisation d'imageur selon ce principe. Un substrat Tx transparent aux ondes térahertz comprend une matrice d'émetteurs térahertz 10. Le substrat est conçu pour être appliqué contre une zone d'analyse 12, par exemple la peau. Le substrat Tx peut être en contact direct avec la surface 12 ou maintenu à un écart donné à l'aide d'un support 14.

Dans ce mode de réalisation, la matrice de pixels émetteurs 10 se trouve sur une face avant du substrat Tx qui est tournée vers la zone 12. L'épaisseur du substrat est en général choisie au plus égale à la moitié de la longueur d'onde du rayonnement à l'intérieur du substrat, ce qui permet de limiter les réflexions internes susceptibles de perturber les pixels voisins.

Les pixels émetteurs selon cette configuration émettent des ondes par les deux faces du substrat. Ainsi, chaque émetteur présente, pour chaque face du substrat, un lobe de puissance d'émission en fonction de l'angle. Le lobe en face avant est plus petit que le lobe en face arrière avec cette configuration, signifiant que l'émetteur est plus efficace par la face arrière (et qu'il est normalement conçu pour être utilisé par la face arrière).

Les émetteurs présentent également des lobes définissant les limites du champ proche. Un lobe de puissance définit un facteur compris entre 0 et 1, alors qu'un lobe de champ proche définit la limite spatiale du fonctionnement en champ proche. La figure 1 illustre un exemple de lobes de champ proche NF. Ces lobes de champ proche sont sensiblement symétriques par rapport au plan de l'imageur et ils ont une amplitude de l'ordre d'une longueur d'onde dans l'air. Leur forme exacte, déterminable par des simulations complexes, dépend de la configuration des antennes et des éléments voisins.

Il s'avère que la nature des éléments qui se trouvent dans le champ proche d'un émetteur térahertz peuvent affecter les caractéristiques de l'onde, en particulier par l'intermédiaire de l'oscillateur de l'émetteur. Selon le cas, l'oscillateur peut subir un décalage d'impédance, de phase, de fréquence, ou d'amplitude. Ces altérations sont répercutées uniformément sur l'onde émise par les deux faces du substrat. Selon la fréquence, certaines altérations ou le dépassement de certains seuils peuvent constituer une signature caractéristique de matériaux ou propriétés recherchés dans la zone analysée, par exemple des tissus cancéreux dans la peau, que l'on peut discriminer par une proportion d'eau plus importante.

L'imageur de la figure 1 est conçu pour utiliser cette propriété de champ proche. L'écart entre le substrat Tx et la zone de mesure 12 est choisi pour que la zone de mesure 12 croise les lobes de champ proche NF en face avant du substrat Tx. L'écart peut être choisi pour que la surface des lobes au croisement avec la zone 12 soit au plus égale à la surface des pixels émetteurs. On assure de cette manière la meilleure couverture de détection.

Un objet aux propriétés particulières 16 été représenté dans la zone d'analyse en contact avec les lobes de champ proche des deuxième et troisième émetteurs. L'objet 16 affecte l'onde émise par ces émetteurs, ce qui est représenté par des lobes en pointillés.

Les ondes émises en face arrière par les émetteurs 10 sont reçues par des récepteurs térahertz respectifs 18 agencés dans une matrice de même pas que la matrice d'émetteurs. La matrice de récepteurs 18 peut être formée sur la face avant d'un substrat Rx ayant les mêmes caractéristiques que le substrat Tx. L'écart entre la matrice de récepteurs et la matrice d'émetteurs est tel que les émetteurs et récepteurs soient couplés en champ proche, si possible de manière que chaque émetteur 10 soit couplé à un récepteur respectif 18 unique. Avec cette configuration, chaque récepteur 18 mesure et restitue les propriétés de l'onde émise par son émetteur 10 respectif, y compris ses éventuelles altérations.

En pratique, si la surface analysée 12 est solide, tous les émetteurs seront plus ou moins perturbés en champ proche. Le système d'imagerie peut être configuré de façon générique pour générer une image de la zone analysée représentant la phase, la fréquence et l'amplitude des ondes en fausses couleurs, ou générer trois images séparées en échelle de gris pour chacun de ces paramètres. Les paramètres peuvent être combinés en un seul avec des coefficients de pondération pour accentuer des propriétés caractéristiques.

Dans l'exemple de la figure 1, les lobes de champ proche NF ont de plutôt bonnes propriétés en ce qu'ils ne dépassent pas la largeur d'un pixel. Dans une telle situation, il suffit de régler les écarts pour que la zone de mesure 12 croise les parties les plus larges des lobes face avant, et pour que les récepteurs 18 se situent dans les parties les plus larges des lobes face arrière, ce qui correspond sensiblement à ce qui est représenté. On obtient ainsi la meilleure sensibilité. Il est par ailleurs souhaitable que les lobes face arrière traversent la plus grande épaisseur de substrat possible, ce qui est obtenu en plaçant les substrats face arrière contre face arrière. Cependant, la présence entre les substrats d'une couche d'indice de réfraction inférieur à celui des substrats (air, vide ou autre) est bénéfique, car elle permet aux substrats de rester indépendants quant à leur contrainte d'épaisseur pour limiter les réflexions internes.

Par ailleurs, dans l'exemple de la figure 1, il n'y a pas de possibilité de diaphonie entre récepteurs, du fait que le lobe face arrière d'un émetteur ne chevauche jamais plusieurs pixels récepteurs. Il n'y a pas non plus de possibilité de diaphonie entre émetteurs, du fait que le lobe face avant ne déborde pas d'un pixel émetteur à l'autre. Cette configuration permet d'utiliser tous les émetteurs et récepteurs simultanément, en « obturateur global ».

Dans le cas général, surtout si on cherche à réduire le pas des pixels, chaque lobe face arrière peut déborder sur plusieurs pixels récepteurs, selon la distance entre les émetteurs et les récepteurs. Pour les lobes face avant, on peut toujours approcher la face avant aussi près que l'on veut de la zone d'analyse 12 pour limiter la surface utile des lobes à la surface des pixels.

Pour éviter les problèmes de diaphonie dans le cas général, il suffit d'activer chaque émetteur en séquence avec son récepteur respectif. Ainsi, même si plusieurs récepteurs voisins voient le lobe de l'émetteur activé, seul le récepteur désigné est activé pour prendre la mesure.

On peut créer une séquence où plusieurs émetteurs à la fois sont activés selon un motif où chaque émetteur activé n'interfère pas avec les récepteurs associés aux autres émetteurs activés.

A la figure 1, chaque substrat comprend un circuit de commande, 20 pour les émetteurs et 22 pour les récepteurs, destiné à gérer la matrice, notamment l'activation des pixels. L'activation d'un pixel émetteur inclut notamment la mise en route d'un oscillateur local du pixel ou la connexion du pixel à un signal d'oscillateur partagé. L'activation d'un pixel récepteur inclut notamment le prélèvement des mesures.

Les pixels émetteurs et récepteurs étant commandés en synchronisme, les signaux nécessaires à la synchronisation peuvent être véhiculés entre les circuits 20 et 22 par une liaison 24 guidée dans le support 14.

Les récepteurs peuvent être de type homodyne, de structure très simple, sans oscillateur, mais capable seulement de fournir une mesure d'amplitude. Si on souhaite exploiter des informations de phase ou de fréquence, on peut utiliser des récepteurs de type hétérodyne. L'article ["A Fully Integrated 320 GHz Coherent Imaging Transceiver in 130 nm SiGe BiCMOS", Chen Jiang et al., IEEE Journal of Solid-State Circuits, Vol. 51, No. 11, November 2016] ainsi que la thèse de Hani Sherry susmentionnée décrivent des mises en œuvre de récepteurs térahertz hétérodynes en technologie des semi-conducteurs.

Les substrats Rx et Tx avec leurs pixels et circuits de commande peuvent être réalisés sous forme de puces de semi-conducteur, par exemple en technologie CMOS. On pourra utiliser des techniques connues d'assemblage puce-sur-puce pour assembler les deux substrats ou puces Rx et Tx avec l'écart souhaité.

Selon un mode de réalisation, les pixels émetteurs et récepteurs sont hexagonaux et agencés selon une matrice en nid d'abeille. Cette configuration en hexagone des pixels est particulièrement bien adaptée à la structure des émetteurs et récepteurs THz envisagés. En effet, ceux-ci peuvent être basés sur une antenne annulaire, comme on le verra ci-après, et la structure hexagonale est plus compacte qu'une structure carrée pour contenir une antenne annulaire. Du fait en outre que la matrice est en nid d'abeille, celle-ci peut loger un plus grand nombre de pixels pour un pas donné entre pixels. Ces caractéristiques combinées permettent d'obtenir une résolution sensiblement plus élevée pour un pas donné qu'une matrice carrée et un meilleur rendu de lignes obliques.

La figure 2 représente une vue de dessus partielle d'un mode de réalisation de matrice de pixels hexagonaux réalisés dans une technologie des semi-conducteurs, par exemple CMOS 65 nm. On a représenté la matrice de pixels récepteurs Rx. La matrice de pixels émetteurs est similaire, puisqu'elle est soumise aux mêmes contraintes, définies par les dimensions des antennes. Les éléments de cette vue sont sensiblement à l'échelle pour un imageur conçu pour travailler à environ 600 GHz, à titre d'exemple. La fréquence de 600 GHz correspond à une longueur d'onde de 0,5 mm dans l'air. Les pixels sont intégrés dans un substrat en silicium, où la longueur d'onde subit une réduction d'un facteur multiplicatif d'environ 0,6, ramenant la longueur d'onde à environ 0,3 mm dans le silicium. Par ailleurs, on accepte de ne travailler que sur la moitié de la longueur d'onde, soit 0,15 mm, car cela permet d'augmenter la résolution d'un facteur 2 avec une perte acceptable de gain. Ainsi, les antennes 50 des émetteurs et récepteurs sont dimensionnées pour travailler avec cette longueur d'onde. Les antennes 50 sont ici annulaires, ce qui implique que leur circonférence moyenne est au moins égale à la longueur d'onde de travail, soit 0,15 mm.

Les anneaux sont réalisés, par exemple, dans la dernière couche de métal de la technologie et ont une largeur de 10 µm, soit un diamètre externe de 64 µm et un diamètre interne de 54 µm.

Par ailleurs, pour éviter la propagation transversale de perturbations électriques par couplage inductif ou capacitif entre pixels, chaque pixel comprend un anneau de garde périphérique 52, anneau qui peut être circulaire ou, ici, hexagonal. L'antenne est centrée dans une zone majoritairement dépourvue de métal dont le diamètre moyen est sensiblement égal à la longueur d'onde de travail (0,15 mm). Ainsi, le bord intérieur de l'anneau de garde est au moins à 38 µm du bord externe de l'anneau d'antenne. L'anneau de garde a par ailleurs une largeur de 30 µm, et est structuré pour respecter un rapport métal/vide préconisé par la technologie. Le pixel a ainsi une largeur de 200 µm entre deux faces opposées de l'hexagone, valeur correspondant au pas selon chacun de trois axes à 0°, 120° et 240°.

La figure 3 est une vue en coupe des pixels de la figure 2. Les pixels sont formés sur la face active d'un substrat semi-conducteur 60, ici en silicium. Les antennes 50, réalisées dans le dernier niveau de métal, affleurent la face supérieure du substrat. Cette face supérieure est normalement recouverte d'une couche de passivation, non représentée. Les anneaux de garde 52, comme cela est représenté, peuvent s'étendre en profondeur à l'aide de plages métalliques empilées dans tous les niveaux de métal de la technologie, sept en CMOS 65 nm, interconnectées par des vias. Les vias peuvent être agencés autour de chaque pixel, selon un pas apte à parfaire la fonction d'écrantage.

Pour limiter les réflexions internes, comme on l'a précédemment indiqué, l'épaisseur du substrat 60 est de 0,15 mm.

Comme on l'a représenté pour une paroi de l'un des anneaux de garde, les plages métalliques peuvent être structurées pour former une cavité 62. La cavité 62 peut loger des pistes conductrices et des composants électroniques servant à l'exploitation des pixels. En effet, la largeur de deux anneaux de garde juxtaposés est de l'ordre de 60 µm, ce qui, en une technologie de 65 nm, offre suffisamment de place pour loger la majorité des conducteurs et composants électroniques requis localement pour exploiter les pixels. Cette configuration permet de réduire au strict minimum les conducteurs métalliques dans la zone vide autour des antennes, qui perturberaient les propriétés optiques.

## Revendications

1. Capteur pour système d'imagerie térahertz en champ proche, comprenant :
• une matrice d'émetteurs (10) d'un rayonnement térahertz configurée de manière que les émetteurs interagissent avec un objet à analyser (12) par effet de champ proche ;
**caractérisé en ce que** chaque émetteur est configuré pour émettre un champ par les deux faces de la matrice, et **en ce que** le capteur comprend en outre :
• une matrice de réception (18) disposée du côté opposé de la matrice d'émetteurs par rapport à l'objet à analyser (12), incluant un récepteur du rayonnement térahertz en face de chaque émetteur, disposé dans le champ proche de l'émetteur.

2. Capteur selon la revendication 1, comprenant :
• un premier substrat plan (Rx) en matériau semi-conducteur transparent aux rayonnements térahertz, ayant une face active sur laquelle sont réalisés les récepteurs (18) dans une technologie des semi-conducteurs ; et
• un deuxième substrat plan (Tx) distinct du premier, en matériau semi-conducteur transparent aux rayonnements térahertz, ayant une face active sur laquelle sont réalisés les émetteurs (10) dans une technologie des semi-conducteurs.

3. Capteur selon la revendication 1, comprenant un circuit de commande (20, 22) configuré pour activer en séquence chaque émetteur avec son récepteur respectif.

4. Capteur selon la revendication 2, dans lequel la face active du deuxième substrat (Tx) est tournée vers l'objet à analyser (12), et une face arrière du deuxième substrat est tournée vers le premier substrat (Rx).

5. Capteur selon la revendication 4, dans lequel la face active du premier substrat (Rx) est tournée du côté opposé au deuxième substrat (Tx), et une face arrière du premier substrat est tournée vers le deuxième substrat.

6. Capteur selon la revendication 5, dans lequel les premier et deuxième substrats sont séparés l'un de l'autre par une couche ayant un indice de réfraction plus faible que celui des substrats.

7. Capteur selon la revendication 2, dans lequel le pas des matrices est au moins égal à la moitié de la longueur d'onde du rayonnement à l'intérieur des substrats et chaque substrat a une épaisseur au plus égale à la moitié de la longueur d'onde du rayonnement à l'intérieur du substrat.

8. Capteur selon la revendication 7, dans lequel les récepteurs et les émetteurs ont une configuration hexagonale et sont agencés selon des matrices en nid d'abeille.

9. Capteur selon la revendication 8, dans lequel chaque récepteur et émetteur comprend :
• une antenne annulaire (50) formée dans un niveau de métal de la face active, la circonférence moyenne de l'antenne étant au moins égale à la moitié de la longueur d'onde du rayonnement térahertz à l'intérieur du substrat ; et
• un anneau de garde (52) entourant l'antenne à la périphérie du récepteur ou émetteur, formé à partir de plages métalliques empilées sur plusieurs niveaux de métal.

10. Capteur selon la revendication 8, dans lequel l'anneau de garde (52) comprend des plages métalliques structurées pour former une cavité (62) abritant des pistes conductrices et des composants électroniques servant à exploiter les récepteurs et émetteurs.

## Patentansprüche

1. Sensor für ein Nahfeld-Terahertz-Bildgebungssystem, der Folgendes umfasst:
• eine Sendermatrix (10) für Terahertzstrahlung, die so konfiguriert ist, dass die Sender durch einen Nahfeldeffekt mit einem zu analysierenden Objekt (12) wechselwirken;
**dadurch gekennzeichnet, dass** jeder Sender zum Senden eines Feldes durch die zwei Flächen der Matrix konfiguriert ist, und dass der Sensor ferner Folgendes umfasst:
• eine Empfangsmatrix (18), die auf der gegenüberliegenden Seite der Sendermatrix in Bezug auf das zu analysierende Objekt (12) angeordnet ist, die einen Empfänger der Terahertzstrahlung gegenüber jedem Sender einschließt, der in dem Nahfeld des Senders angeordnet ist.

2. Sensor nach Anspruch 1, der Folgendes umfasst:
• ein erstes planares Substrat (Rx) aus einem für Terahertzstrahlung durchlässigen Halbleitermaterial, das eine aktive Fläche aufweist, auf der die Empfänger (18) in einer Halbleitertechnologie hergestellt sind; und
• ein zweites planares Substrat (Tx), das sich von dem ersten unterscheidet, aus einem für Terahertzstrahlung durchlässigen Halbleitermaterial, das eine aktive Fläche aufweist, auf der die Sender (10) in einer Halbleitertechnologie hergestellt sind.

3. Sensor nach Anspruch 1, der eine Steuerschaltung (20, 22) umfasst, die zum sequenziellen Aktivieren jedes Senders mit seinem jeweiligen Empfänger konfiguriert ist.

4. Sensor nach Anspruch 2, wobei die aktive Fläche des zweiten Substrats (Tx) dem zu analysierenden Objekt (12) zugewandt ist und eine hintere Fläche des zweiten Substrats dem ersten Substrat (Rx) zugewandt ist.

5. Sensor nach Anspruch 4, wobei die aktive Fläche des ersten Substrats (Rx) der dem zweiten Substrat (Tx) gegenüberliegenden Seite zugewandt ist und eine hintere Fläche des ersten Substrats dem zweiten Substrat zugewandt ist.

6. Sensor nach Anspruch 5, wobei das erste und das zweite Substrat durch eine Schicht voneinander getrennt sind, die einen niedrigeren Brechungsindex als der der Substrate aufweist.

7. Sensor nach Anspruch 2, wobei der Abstand der Matrizen wenigstens gleich der Hälfte der Wellenlänge der Strahlung innerhalb der Substrate ist und jedes Substrat eine Dicke aufweist, die höchstens gleich der Hälfte der Wellenlänge der Strahlung innerhalb des Substrats ist.

8. Sensor nach Anspruch 7, wobei die Empfänger und die Sender eine sechseckige Konfiguration aufweisen und in Matrizen in Wabenform eingerichtet sind.

9. Sensor nach Anspruch 8, wobei jeder Empfänger und Sender Folgendes umfasst:
• eine ringförmige Antenne (50), die in einer Metallebene der aktiven Fläche ausgebildet ist, wobei der durchschnittliche Umfang der Antenne wenigstens gleich der Hälfte der Wellenlänge der Terahertzstrahlung innerhalb des Substrats ist; und
• einen Schutzring (52), der die Antenne an der Peripherie des Empfängers oder Senders umgibt, der aus metallischen Bereichen ausgebildet ist, die auf mehreren Metallebenen gestapelt sind.

10. Sensor nach Anspruch 8, wobei der Schutzring (52) metallische Bereiche umfasst, die zum Ausbilden eines Hohlraums (62) strukturiert sind, der Leiterbahnen und elektronische Komponenten, die zum Betreiben von Empfängern und Sendern dienen, aufnimmt.

## Claims

1. A sensor for a near-field terahertz imaging system, comprising:
an array of terahertz radiation transmitters (10) configured such that the transmitters interact with an object to be analyzed (12) by near field effect;
wherein each transmitter is configured to emit a field from two faces of the array; and the sensor further comprises:
a receiver array (18) arranged on the opposite side of the transmitter array from the object to be analyzed (12), including a terahertz radiation receiver opposite each transmitter, located in the near field of the transmitter.

2. The sensor according to claim 1, comprising:
a first planar substrate (Rx) of semiconductor material transparent to terahertz radiation, having an active face on which the receivers (18) are realized in semiconductor technology; and
a second planar substrate (Tx) distinct from the first substrate, made of semiconductor material transparent to terahertz radiation, having an active face on which the transmitters (10) are realized in semiconductor technology.

3. The sensor according to claim 1, comprising a control circuit (20, 22) configured to activate in sequence each transmitter with its respective receiver.

4. The sensor according to claim 2, wherein the active face of the second substrate (Tx) faces the object to be analyzed (12), and a back face of the second substrate faces the first substrate (Rx).

5. The sensor according to claim 4, wherein the active face of the first substrate (Rx) faces away from the second substrate (Tx), and a back face of the first substrate faces the second substrate.

6. The sensor according to claim 5, wherein the first and second substrates are separated from each other by a layer having a lower refractive index than the substrates.

7. The sensor according to claim 2, wherein the pitch of the arrays is at least half the wavelength of the radiation within the substrates and each substrate has a thickness of at most half the wavelength of the radiation within the substrate.

8. The sensor according to claim 7, wherein the receivers and transmitters have a hexagonal configuration and are arranged in honeycomb matrices.

9. The sensor according to claim 8, wherein each receiver and transmitter comprises:
an annular antenna (50) formed in a metal level of the active face, the average circumference of the antenna being at least half the wavelength of the terahertz radiation within the substrate; and
a guard ring (52) surrounding the antenna at the periphery of the receiver or transmitter, formed from metal patterns stacked through several levels of metal.

10. The sensor according to claim 8, wherein the guard ring (52) comprises metal patterns structured to form a cavity (62) housing conductor tracks and electronic components for operating the receivers and transmitters.
